# EUROPEAN PATENT SPECIFICATION

(11) **EP 4 422 520 B1**
(45) Date of publication and mention of the grant of the patent: **27.05.2026**
(21) Application number: 23711311.3
(22) Date of filing: 21.02.2023
(51) Int. Cl.: A61B 17/122, A61B 17/00, A61B 17/128, A61B 90/00

(54) **HEMOSTASIS CLIP RELEASE MECHANISM**
HÄMOSTASECLIPFREISETZUNGSMECHANISMUS
MÉCANISME DE LIBÉRATION DE PINCE D'HÉMOSTASE

(30) Priority: 11.04.2022 US 202263362801 P
(43) Date of publication of application: 04.09.2024
(73) Proprietor: Boston Scientific Scimed, Inc., Maple Grove, MN 55311 (US)
(72) Inventor: SMITH, Paul, Smithfield, Rhode Island 02917 (US); GREENE, Eric, Wayland, Massachusetts 01778 (US); ESTEVEZ, Ramon, Lowell, Massachusetts 01852 (US); ADAMS, Matthew Bradley, Harvard, Massachusetts 01451 (US)
(74) Representative: Vossius & Partner Patentanwälte Rechtsanwälte mbB
(86) International application number: PCT/US2023/013530
(87) International publication number: WO 2023/200527

(56) References cited:
- US-A1- 2020 397 445

## Description

### Field

The present disclosure relates to endoscopic devices and, in particular, relates to endoscopic clipping devices for treating tissue along the gastrointestinal tract.

### Background

Physicians have become more willing to perform aggressive interventional and therapeutic endoscopic gastrointestinal (GI) procedures, which may increase the risk of perforating the wall of the GI tract or may require closure of the GI tract wall as part of the procedure. Such procedures may include, for example, the removal of large lesions, tunneling under the mucosal layer of the GI tract to treat issues below the mucosa, full thickness removal of tissue, treatment of issues on other organs by passing outside of the GI tract, and endoscopic treatment/repair of post-surgical issues (e.g., post-surgical leaks, breakdown of surgical staple lines, and anastomotic leaks).

Currently, tissue may be treated via endoscopic closure devices including through-the scope clips or over-the-scope clips. Over-the-scope clips may be particularly useful for achieving closure of larger tissue defects. These endoscopic closure devices can save costs for the hospital and may provide benefits for the patient. In some cases, however, current endoscopic closure devices may be difficult to use, time consuming to position, or insufficient for certain perforations, conditions and anatomies. For example, current over-the-scope clips generally require launching of the clip from a position in which the clip itself is not visible to the operator. That is, prior to clipping the operator may view the target tissue to be clipped and, based on this visualization of the target tissue may determine that the distal end of the device and the clip are in a desired position relative to the target tissue. Based on the observation of the target tissue, the operator then deploys the clip without being able to see the clip itself until it is deployed. Once deployed, such current over-the scope clips are generally incapable of being repositioned.

US 2020/397445 A1 discloses a system for compressing body tissue including a clip movable from a closed position to an open position. A clip deployment device has a first clip engagement member and a second clip engagement member engageable with the clip, the first and second clip engagement members are movable between first and second positions to controllably move the clip from the closed position to the open position.

### Summary

The present invention relates to a clipping system for treating tissue as defined in the appended claims 1 to 15. The system according to claim 1 includes an adapter, a clip and a first extending member. The adapter includes a proximal portion configured to be mounted over a distal end of an insertion device and a distal portion extending distally from the proximal portion. The clip is configured to be mounted over the distal portion of the adapter. The clip includes first and second jaws connected to one another such that the first and second jaws are movable between an insertion configuration, in which the first and second jaws extend about opposing portions of the distal portion of the adapter and are separated from one another to receive tissue therebetween, and an initial deployed configuration, in which the clip is moved distally off of the adapter so that the first and second jaws are drawn toward one another to grip tissue therebetween. The first and second jaws are biased toward the initial deployed configuration.

The first extending member is releasably coupled to the clip and movably connected to the adapter. The first extending member includes a connector at a distal end thereof configured to releasably engage an opening extending through the first jaw such that a longitudinal movement of the first extending member relative to the adapter moves the clip between the insertion configuration, the initial deployed configuration and toward a review configuration, in which the adapter is permitted to be withdrawn proximally away from the clip while the first extending member remains coupled to the clip to enhance visual observation of the clip, the first extending member being operable to retract the clip proximally over the adapter so that the clip is forced open as the clip is retracted over the adapter freeing the clip from tissue on which it had been clipped. The connector includes a deformable portion configured to deform to release the first extending member from the first jaw toward a final deployed configuration when a distal force exerted on the first extending member relative to the clip exceeds a predetermined threshold value. The first extending member is proximally withdrawable from the clip upon deformation of the connector.

In an embodiment, the connector includes a pair of wings extending radially away from the distal end of the first extending member, the pair of wings configured to shear off of the distal end of the first extending member as the pair of wings are deformed toward the final deployed configuration.

In an embodiment, the system further includes an insert configured to be received and affixed within the opening of the first jaw, the insert including a hole extending longitudinally therethrough and a recess extending radially outward from the hole, the recess sized, shaped and configured to receive the pair of wings therein.

In an embodiment, a radially innermost point at which each of the wings is connected to the distal end of the first extending member has a thickness smaller than a remaining portion of the wings.

In an embodiment, a distal surface of the pair of wings includes a tapered portion angled at a non-perpendicular angle relative to a longitudinal axis of the first extending member, the tapered portion configured to engage a sharp cutting edge along a surface of the recess when pressed distally thereagainst.

In an embodiment, the opening of the first jaw extending through the clip from a first surface facing toward the adapter to a second surface facing away from the adapter, the opening including a first portion configured to engage the connector and a second portion configured as a slot extending from the first portion to an exterior of the clip.

In an embodiment, the connector of the first extending member includes a head portion and a shaft extending proximally therefrom, the shaft being sized, shaped and configured to engage the first portion of the opening, a width of the slot being smaller than the shaft to prevent a passage of the connector therethrough.

In an embodiment, the distal end of the first extending member is biased laterally away from a longitudinal axis of the adapter so that, when the connector is received within the first portion of the opening the distal end of the first extending member is constrained toward a laterally inward position, and when the connector is pushed distally out of the first portion of the opening, the distal end reverts towards its biased laterally outward position so that a remaining length of the first extending member extending proximally from the connector is passed through the slot of the second portion of the opening to an exterior of the clip.

In an embodiment, the connector including a deformable arm extending laterally from the shaft to engage the first surface of the clip when the shaft is received within the first portion of the opening to move the clip between the insertion configuration, the initial deployed configuration and the review configuration, the deformable arm configured to deform to release the connector from the first portion of the opening when a distal force applied to the first extending member relative to the clip exceeds the predetermined threshold value.

In an embodiment, the system further includes a second extending member releasably coupled to the clip and movably connected to the adapter, the second extending member including a further connector at a distal end thereof configured to releasably engage an opening extending through the second jaw such that a longitudinal movement of the second extending member relative to the adapter moves the clip between the insertion configuration, the initial deployed configuration and the review configuration, the further connector of the second extending member including a deformable portion configured to deform to release the second extending member from the second jaw toward the final deployed configuration when a distal force exerted on the second extending member relative to the clip exceeds a predetermined threshold value.

The system according to claim 11 includes an adapter, a clip and a first extending member. The adapter includes a proximal portion configured to be mounted over a distal end of an insertion device and a distal portion extending distally from the proximal portion; The clip is configured to be mounted over the distal portion of the adapter. The clip includes first and second jaws connected to one another such that the first and second jaws are movable between an insertion configuration, in which the first and second jaws extend about opposing portions of the distal portion of the adapter and are separated from one another to receive tissue therebetween, and an initial deployed configuration, in which the clip is moved distally off of the adapter so that the first and second jaws are drawn toward one another to grip tissue therebetween. The first and second jaws are biased toward the initial deployed configuration.

The first extending member is releasably coupled to the clip and movably connected to the adapter. The first extending member includes a distal end biased toward a coiled configuration and configured to releasably engage a portion of an opening extending through the first jaw such that a longitudinal movement of the first extending member relative to the adapter moves the clip between the insertion configuration, the initial deployed configuration and toward a review configuration, in which the adapter is permitted to be withdrawn proximally away from the clip while the first extending member remains coupled to the clip to enhance visual observation of the clip, the first extending member being operable to retract the clip proximally over the adapter so that the clip is forced open as the clip is retracted over the adapter freeing the clip from tissue on which it had been clipped, the coiled configuration of the first extending member configured to unravel when subject to a force exceeding a predetermined threshold value so that the first extending member is disengaged from the first jaw toward a final deployed configuration.

In an embodiment, the system further includes an insert configured to be received and affixed within the opening of the first jaw, the insert including a curved opening extending thereinto, the curved opening configured to receive and engage the distal end of the first extending member.

In an embodiment, the distal end of the first extending member is reinforced with a compressible rod.

In an embodiment, the curved opening includes an angled edge extending therealong.

In an embodiment, the system further includes a second extending member releasably coupled to the clip and movably connected to the adapter, the second extending member including a distal end biased toward a coiled configuration and configured to releasably engage a portion of an opening extending through the second jaw such that a longitudinal movement of the second extending member relative to the adapter moves the clip between the insertion configuration, the initial deployed configuration and the review configuration, the coiled configuration of the first extending member configured to unravel when subject to a force exceeding a predetermined threshold value so that the second extending member is disengaged from the second jaw toward the final deployed configuration.

In addition, the present disclosure includes a non-claimed method for treating tissue which includes inserting a clip to a target area in a body lumen via an endoscope, the clip mounted over a distal end of the endoscope, via an adapter, in an open insertion configuration in which jaws of the clip are separated from one another; drawing tissue into a channel of the adapter and between jaws of the clip; moving the clip from the open insertion configuration toward an initial deployed configuration by releasing a tension along extending members so that the clip is moved distally off of the adapter and the jaws revert to a biased closed configuration in which the jaws extend toward one another to grip the tissue received therebetween; drawing the endoscope proximally away from the clip, while the clip remains coupled to the extending members, toward a review configuration in which a visualization of the clip via the endoscope is enhanced; and moving the extending members distally relative to the clip until a force exerted thereon exceeds a predetermined threshold value so that connectors at distal ends of the extending members deform to release the extending members from the clip toward a final deployed configuration.

In an embodiment, when it is determined that the clip requires repositioning, drawing the extending members proximally relative to the endoscope until the clip is drawn proximally over the adapter toward the open insertion configuration, and repositioning the clip over the target tissue.

In an embodiment, each of the connectors includes a pair of wings extending radially away from a distal end of the extending members so that the pair of wings shear off of the distal end of the extending members as the pair of wings are deformed toward the final deployed configuration.

In an embodiment, when the extending members are moved toward the final deployed configuration, the extending members are moved distally relative to an insert affixed within openings extending through jaws of the clip, the insert including a hole extending longitudinally therethrough and a recess extending radially outward from the hole, the pair of wings received within the recess.

In an embodiment, when the extending members are moved distally relative to the insert toward the final deployed configuration, a distal surface of the pair of wings including a tapered portion angled at a non-perpendicular angle relative to a longitudinal axis of one of the extending members engages a sharp cutting edge along a surface of the recess when pressed distally thereagainst so that the sharp cutting edge shears the pair of wings off the distal ends of the extending members.

### Brief Description

Fig. 1 shows a longitudinal side view of a tissue clipping system according to an exemplary embodiment of the present disclosure, in an insertion configuration;
Fig. 2 shows a cross-sectional view of a connector received within an opening of an insert according to the system Fig. 1;
Fig. 3 shows a perspective view of a clip according to the system of Fig. 1;
Fig. 4 shows a break-away perspective view of the insert according to the system of Fig. 1;
Fig. 5 shows a longitudinal side view of the system according to Fig. 1, in a review configuration;
Fig. 6 shows a cross-sectional view of an extending member moved distally relative to the insert during initiation of a final deployment configuration according to the system of Fig. 1;
Fig. 7 shows a cross-sectional view of wings of the connector being sheared off of a distal end of the extending member during a final deployment of the clip, according to the system of Fig. 1;
Fig. 8 shows a withdrawal of the distal end of the extending member from the insert during the final deployment of the clip, according to the system of Fig. 1;
Fig. 9 shows a longitudinal side view of the system according to the system of Fig. 1, in the final deployed configuration;
Fig. 10 shows a cross-sectional side view of an insert and extending members engaged with one another according to another exemplary embodiment of the present disclosure;
Fig. 11 shows a cross-sectional side view of the insert and extending members disengaged from one another according to the embodiment of Fig. 10;
Fig. 12 shows a longitudinal side view of a distal portion of a clipping system according to another exemplary embodiment of the present disclosure, in an insertion configuration;
Fig. 13 shows a longitudinal side view of the distal portion of the system according to Fig. 12, in a review configuration;
Fig. 14 shows a longitudinal side view of the distal portion of the system according to Fig. 12, during a final deployment process;
Fig. 15 shows an enlarged perspective view of a portion of a clip of the system according to Fig. 12, including an opening extending therethrough;
Fig. 16 shows a longitudinal side view of a distal end of an extending member according to the system o Fig. 12;
Fig. 17 shows a cross-sectional view of a portion of a clip and an extending member engaged therewith according to another exemplary embodiment of the present disclosure;
Fig. 18 shows a cross-sectional view of a portion of the clip deformed to permit release of the extending member therefrom according to the embodiment of Fig. 17;
Fig. 19 shows a cross-sectional view of the extending member disengaged from the clip according to the embodiment of Fig. 17;
Fig. 20 shows a cross-sectional view of a portion of a clip deformed to permit release of an extending member therefrom according to another exemplary embodiment of the present disclosure; and
Fig. 21 shows a cross-sectional view of the extending member disengaged from the clip according to the embodiment of Fig. 20.

### Detailed Description

The present disclosure may be further understood with reference to the following description and the appended drawings, wherein like elements are referred to with the same reference numerals. The present disclosure relates to a clipping system and, in particular, relates to an over-the-scope endoscopic clipping system, in which an initial placement of a clip may be viewed and adjusted prior to a final deployment thereof. Exemplary embodiments of the present disclosure comprise a clip mountable over a distal end of an endoscope via an adapter and releasably coupled to extending members so that the clip may be moved relative to the adapter between an insertion configuration, an initial deployed configuration, and a review configuration, in which the clip can be viewed via the endoscopic viewing system prior to being finally deployed.

According to an exemplary embodiment, a distal end of each of the extending members is coupled to a corresponding jaw of the clip via a coupling mechanism configured to release to disengage the clip when subject to a force exceeding a predetermined threshold force. In exemplary embodiments, during final deployment of the clip, the extending members may be pushed or pulled relative to the clip until a force exerted thereon exceeds the predetermined threshold force, deforming and/or breaking the coupling mechanism so that the extending members are released from the clip and may be removed from the body, leaving the clip clipped over a target tissue.

While the extending members remain coupled to the clip, however, the extending members may be moved longitudinally relative to the endoscope to move the clip between the insertion configuration, the initial deployed configuration and the review configuration. In the insertion configuration, the clip is mounted over the adapter in a proximal position maintained in the insertion configuration ready to receive tissue between jaws thereof while the clip's position minimizes its occlusion of the field of view of the endoscopic vision system. The insertion configuration is configured to facilitate insertion of the endoscope to a target site adjacent to tissue to be clipped while the system allows the clip to be deployed and clipped over tissue in an initial deployed configuration. The device permits the endoscope to be withdrawn proximally away from the clip and the tissue over which it is clipped while the clip remains coupled to the device in a review configuration.

As the endoscope is withdrawn proximally while the clip remains in place over the target tissue, the field of view of the vision system of the endoscope widens to show the clip and the tissue clipped thereby so that the operator can determine whether the position of the clip is desirable or in need of adjustment. If the operator determines that the clip is positioned as desired, the clip is deployed by releasing the clip from the clasps of the extending members and left in place clipped over the target tissue. If the operator determines that the position of the clip needs adjustment, the endoscope and the adapter coupled thereto are moved distally to a position adjacent to the clip. The clip is then drawn proximally over the adapter to reopen the clip which is drawn proximally over the distal end of the adapter forcing the clip to open against its natural bias as the clip slides proximally back over the adapter to return to the insertion configuration. After the clip has been removed from the tissue and returned to the insertion configuration, the operator can re-position the endoscope and device as desired, draw target tissue into the adapter (e.g., under suction or a grasper applied via a working channel of the endoscope) and once more deploy the clip from the adapter over the target tissue in the initial deployed position.

The endoscope is then withdrawn proximally once again as the clip remains coupled to the device so that the device moves again into the review configuration. The position of the clip and the clipped tissue are again observed and, this process may be repeated until the clip is positioned as desired. When the operator sees that the tissue over which the clip is closed is the desired portion of tissue, the operator may initiate the final deployment process, exerting a force on the extending members relative to the clip so that the extending members are deformed to disengage the clip. The extending members may then be removed from the body, leaving the clip clipped over the target tissue. It will be understood by those of skill in the art that terms proximal and distal, as used herein, are intended to refer to a direction toward and away from, respectively, a user of the device.

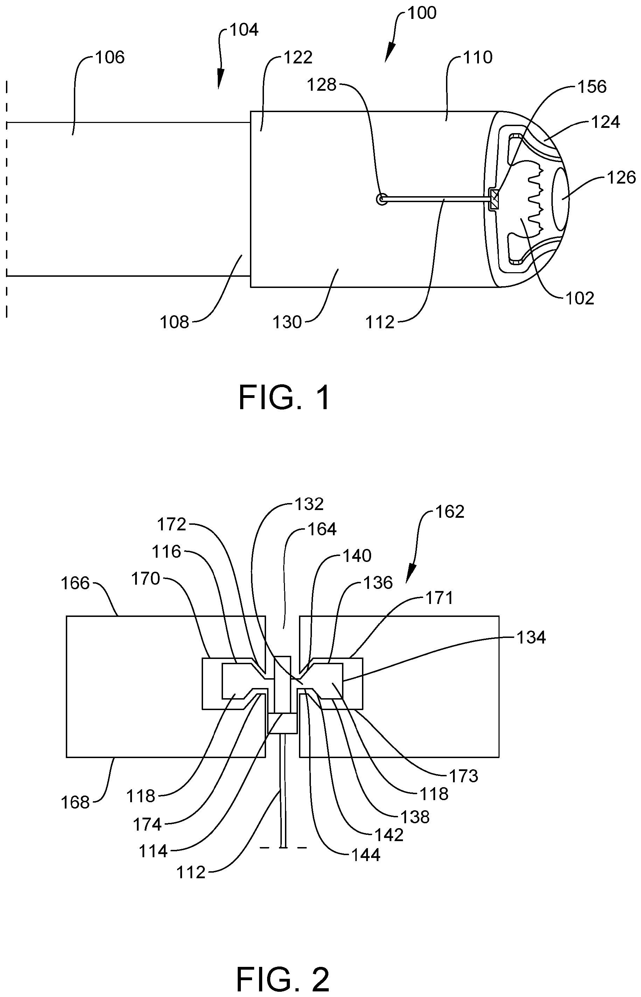

As shown in Figs. 1 - 9, a clipping system 100 for treating tissue defects and/or perforations according to an exemplary embodiment comprises a clip 102 configured to be inserted through, for example, a body lumen to a target area to clip target tissue (e.g., tissue of a wall of the lumen). The clip 102 is insertable to the target area via an insertion device 104 such as, for example, an endoscope 106. The clip 102 is configured to be coupled to a distal end 108 of the endoscope 106 via an adapter 110, which is mounted over the distal end 108 of the endoscope 106. The clip 102 is configured to be moved relative to the adapter 110 and the endoscope 106 between an insertion configuration, an initial deployed configuration in which the clip 102 is closed over target tissue, and a review configuration via extending members 112, to which the clip 102 is releasably coupled.

In one exemplary embodiment, a distal end 114 of each of the extending members 112 includes a connector 116 configured to engage a correspondingly sized, shaped and configured portion of the clip 102. The connector 116 includes a pair of wings 118 extending radially outward from the distal end 114. The wings 118 are configured to be sheared off when the extending members 112 are moved distally relative to the clip 102 via a force exceeding a predetermined threshold force, so that, the distal end 114 is released from the clip 102 and is removable therefrom toward in a final deployed configuration.

While the pair of wings 118 remain connected to the distal end 114, however, longitudinal movement of the extending members 112 relative to the endoscope 106 moves the clip 102 between the insertion configuration, the initial deployed configuration, and the review configuration. In the insertion configuration, as shown in Fig. 1, the clip 102 is mounted over the adapter 110 with jaws 120 separated from one another to receive tissue therebetween. To move the clip 102 from the insertion configuration toward the initial deployed configuration, the extending members 112 are moved distally relative to the endoscope 106, permitting the clip 102 to be moved distally off of the adapter 110 toward a closed configuration, in which the jaws 120 are moved toward one another to grip tissue that has been drawn into the adapter 110.

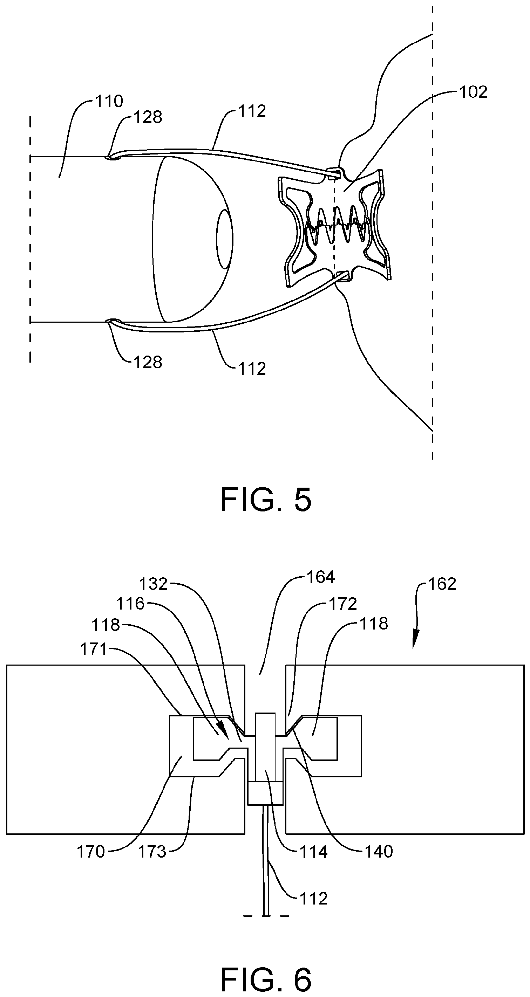

Upon clipping of the tissue via the jaws 120 in the initial deployed configuration, the clip 102 may be moved toward the review configuration, as shown in Fig. 5, by moving the extending members 112 distally away from the endoscope 106 (or drawing the endoscope 106 proximally relative to the extending members 112) so that the clip 102 is distanced from the adapter 110, while remaining tethered to the insertion device 104 via the extending members 112. This widens the field of view of the endoscope vision system relative to the clip 102 and the target tissue and allows for some movement of the endoscope 106 relative to the clip 102 to enable more extensive observation of the placement and/or position of the clip 102 relative to the target tissue. As described below, if the user determines the position of the clip 102 is incorrect or sub-optimal, the user may move the endoscope 106 distally to a position adjacent to the clip 102 and then retract the clip 102 back over the distal end of the adapter 110 toward the open insertion configuration.

The user may then reposition the endoscope 106 and the clip 102 and repeat these steps so that the placement and/or position of the clip 102 relative to a target tissue may be adjusted, as desired, prior to a final deployment of the clip 102. That is, if the operator determines in the review configuration that the clip 102 is not positioned as desired, the clip 102 may be re-opened and removed from the tissue so that the device can be re-positioned until the clip 102 is closed over the desired portion of tissue. Once it is determined that the clip 102 has been clipped over the desired tissue, the extending members 112 are moved distally relative to the clip 102 so that the wings 118 of the distal end 114 are pressed distally against a portion of the clip 102 within which they are received. The extending members 112 are moved distally until a force exerted on the wings 118 exceeds the predetermined threshold force, causing the wings 118 to be sheared off of the distal end 114, thereby releasing the distal end from the clip 102 and allowing the extending members 112 to be removed from the clip 102 so that the clip 102 remains clipped over the target tissue in the final deployed configuration.

As discussed above and as shown in Fig. 1, the insertion device 104 may include any standard endoscope 106. The clip 102 may be mounted to the endoscope 106 via the adapter 110, which is sized, shaped and configured to be mounted over the distal end 108 of the endoscope 106. As will be understood by those of skill in the art, the endoscope 106 is configured to be inserted through a body lumen to a target area within the lumen and thus, must be sufficiently flexible to navigate through even tortuous paths of the body lumen. The adapter 110 extends from a proximal end 122 configured to be mounted to the distal end 108 of the endoscope 106 to a distal end 124 and includes a channel 126 extending therethrough. The adapter 110 may be mounted to the endoscope 106 via, for example, a friction fit, so that the channel 126 is substantially longitudinally aligned with a channel of the endoscope 106. Thus, tissue is viewable through the channel 126 via an optical system of the endoscope 106. In another embodiment, the adapter 110 may also be formed of a transparent material to enhance a visibility of the tissue.

In one embodiment, the adapter 110 includes a pair of holes 128 extending longitudinally through a wall thereof. The holes 128 are configured to slidably receive the extending members 112 therethrough. An outer diameter of a distal portion 130 of the adapter 110 is sized, shaped and configured to receive the clip 102 thereover, in the insertion configuration. In one exemplary embodiment, the distal portion 130 of the adapter 110 tapers from a maximum diameter at the proximal end 122 toward a minimum diameter at the distal end 124 so that the bias of the clip 102 toward the initial deployed configuration (drawing the jaws 120 toward one another) urges the clip 102 distally over the adapter 110 as the jaws 120 gradually close due to the reducing diameter of the adapter 110 as the clip 102 moves distally thereover.

The clip 102, however, remains mounted over the adapter 110 in the open insertion configuration (i.e., at a proximal position on the adapter 110), with the jaws 120 separated from one another, while a sufficient proximally directed tension is applied to the extending members 112 (i.e., while the extending members 112 hold the clip 102 in place). If this tension is removed from the extending members 112 and the extending members 112 are extended distally while the clip 102 moves over the adapter 110, the natural bias of the clip 102 draws the jaws 120 toward one another pushing the clip 102 distally over the tapered surface of the adapter 110 until the clip 102 slides distally off of the adapter 110.

In one embodiment, the insertion device 104 includes a pair of extending members 112. Each extending member 112 extends longitudinally along or within the endoscope 106 and passes radially out of the holes 128 of the adapter 110 so that distal portions of the extending members 112 are longitudinally movable over the exterior surface of the adapter 110. Each of the extending members 112 extends from a proximal end accessible to the user via, for example, an actuator, to the distal end 114. The extending members 112 may be moved longitudinally relative to the endoscope 106 to move the clip 102, which is coupled thereto, toward and away from the distal end 108 of the endoscope 106 between the insertion configuration, the initial deployed configuration, and the review configuration.

According to an exemplary embodiment, as shown in Fig. 2, the connector 116 of each of the extending members 112 includes a pair of wings 118 extending radially outward from the distal end 114, along a plane substantially perpendicular to a longitudinal axis of the extending member 112. The wings 118, in one embodiment, are molded over the corresponding distal end 114 so that, when the pair of wings 118 are pressed distally against a portion of the clip 102, a proximal force exerted on the wings 118 by the clip 102 shears the wings 118 off of the distal end 114. In an exemplary embodiment, a thickness of each of the wings 118 (a dimension of the wing 118 in a direction perpendicular to a longitudinal (proximal to distal) axis of the adapter 110) at a radially innermost point 132 at which each of the wings 118 contacts the distal end 114 is smaller than a thickness of the same wing 118 at a radially outermost point 134 of the wing 118 to facilitate the shearing off of the wings 118 at the radially innermost points 132. That is, a distance between a distal surface 136 and a proximal surface 138 of each of the wings 118 at the innermost point 132 is smaller than a distance between the distal and proximal surfaces 136, 138 at the radially outermost point 134.

In this embodiment, the distal surface 136 of each of the wings 118 includes an angled surface 140 tapering toward the distal end 114. In other words, the angled surface 140 extends at a non-perpendicular angle relative to a longitudinal axis of the extending member 112. The proximal surface 138 includes a groove 142 extending thereinto (i.e., toward the distal surface 136) at the radially innermost point 132. The groove 142, however, includes a flat 144 extending substantially perpendicularly relative to the longitudinal axis of the extending member 112.

Although the exemplary embodiments show and describe connectors 116 including a pair of wings 118, it will be understood by those of skill in the art that a connector 116 may include any of a variety of radially extending features for engaging a corresponding portion of the clip 102. In one alternate embodiment, rather than wings 118, the connector 116 may include a circumferential engaging element including, for example, slits extending thereabout configured to be sheared off when a proximal force exceeding a predetermined threshold force is exerted thereon by the clip 102 in response to a distally directed force applied to the corresponding extending member 112. Similarly to the wings 118, a circumferentially extending engaging element may extent from the distal end 114 along a plane substantially perpendicular to the longitudinal axis of the extending members 112.

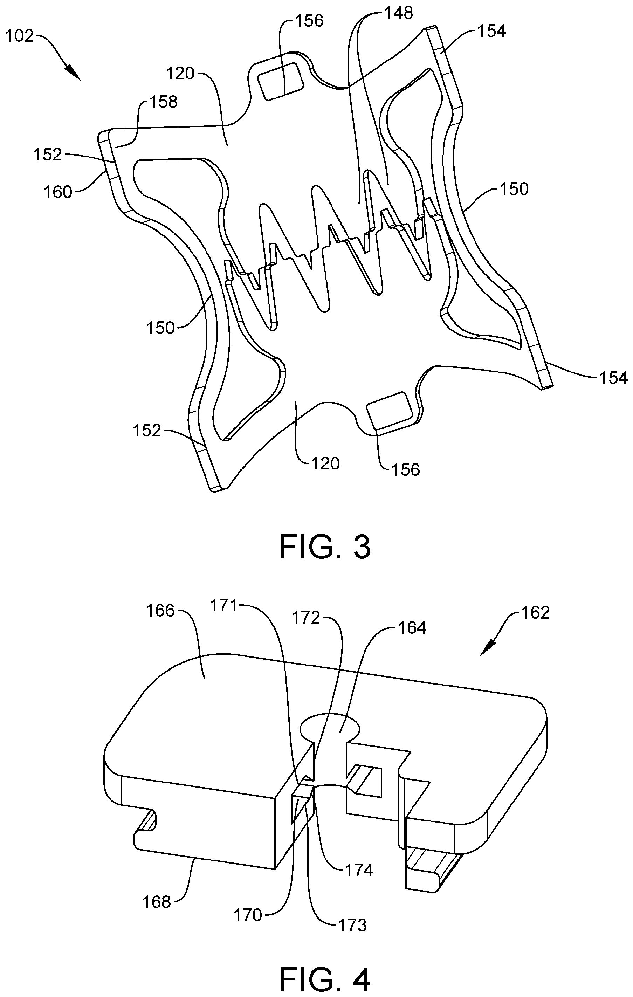

As shown in Fig. 3, the clip 102 includes a pair of jaws 120 including gripping features 148 such as, for example, teeth, for gripping target tissue therebetween. The jaws 120 of one embodiment are connected to one another via hinges 150. In one embodiment, each of the jaws 120 extends along a curve from a first end 152 to a second end 154 so that a first one of the hinges 150 connects the first ends 152 of the jaws 120 to one another while a second one of the hinges 150 connects the second ends 154 of the jaws 120 to one another. In one embodiment, the hinges 150 are spring biased, biasing the jaws 120 toward the initial deployed configuration in which the jaws 120 are closed so that the griping features 148 of a first one of the jaws 120 contact the gripping features 148 of a second one of the jaws 120. In particular, in the initial deployed configuration, the jaws 120 extend toward one another so tissue that had been drawn between the jaws 120 is gripped between the jaws 120 via, for example, the gripping features 148.

However, when the clip 102 is mounted over the adapter 110 in the insertion configuration, the jaws 120 extend about the opposing portions of the adapter 110 with an exterior surface of the adapter 110 maintaining the clip 102 open with the jaws 120 separated from one another so that the target tissue may be drawn into the adapter 110 between the jaws 120. When the clip 102 is moved distally of the adapter 110, the clip 102 is freed to close under the natural bias of the hinges 150 over any tissue that had been drawn into the adapter 110. It will be understood by those of skill in the art that the hinges 150 and/or jaws 120 of the clips 102 may be formed of any of a variety of materials so long as the hinges 150 bias the jaws 120 toward the initial deployed configuration, as described above and so that the bias is sufficiently strong to maintain the clip 102 in position clipped over target tissue after the clip has been finally deployed. In one example, portions of the clip 102 (e.g., the hinges 150) are formed of a shape memory alloy such as, for example, Nitinol to provide and/or add to the bias toward the closed configuration.

Each of the jaws 120 of this embodiment also includes an opening 156 extending therethrough from a first surface 158 of the clip 102, which faces toward the adapter 110, to a second surface 160 of the clip 102, which faces away from the adapter 110. Each opening 156 is configured to releasably engage the connector 116 of a corresponding one of the extending members 112. In one embodiment, each opening 156 is configured to directly engage the wings 118 of a corresponding one of the connectors 116. In this embodiment, each of the openings 156 includes, for example, a radially extending recess within which the wings 118 of the corresponding extending member 112 may be received. To accommodate the radially extending recess, a portion of each of the jaws 120 surrounding the opening 156 may have a greater thickness (e.g., distance between the first and second surfaces 158, 160) than a remaining portion of the clip 102.

In another exemplary embodiment, as shown in the figures, each of the openings 156 may be configured to receive an insert 162 configured to engage one of the connectors 116, as described above. In particular, as shown in Fig. 4, the insert 162 may be sized and shaped to fit within the opening 156 and may be welded, molded, mechanically coupled, or adhered to the clip 102 within the opening 156. The insert 162 includes a hole 164 extending longitudinally therethrough from a first surface of the 166 of the insert 162 to a second surface 168 of the insert 162 such that, when the insert 162 is received within the opening 156, the first surface 166 faces toward the adapter 110 and the second surface 168 faces away from the adapter 110. The insert 162 further includes a recess 170 extending radially outward from the hole 164. The recess 170 of this embodiment is sized and shaped to correspond to the wings 118 of the extending member 112 that is received therein. In one embodiment, the insert 162 includes a pair of recesses 170 each recess 170 being configured to accommodate a corresponding one of the wings 118 of the extending member 112. In another embodiment, the recess 170 extends circumferentially about the hole 164 to accommodate the wings of two or more connectors 116.

A distal surface 171 of the recess 170 of one embodiment includes a sharp cutting edge 172 which, when the wings 118 are pushed distally thereagainst (via a distally directed force applied to the corresponding extending member 112), engages the angled surface 140 of the distal surface 136 at the innermost point 132 of the corresponding wing 118 to facilitate the shearing of the wings 118 at the innermost point 132. A proximal surface 173 of the recess 170, on the other hand, includes a flat surface 174 configured to engage the flat 144 at the innermost point 132 along the proximal surface 138 of the wings 118 so that, even when the wings 118 are drawn proximally thereagainst (e.g., via a proximally directed force applied to the corresponding extending member 112), the wings 118 will not be sheared off. It will be understood by those of skill in the art that the corresponding flat surfaces 174, 144 prevent the inadvertent shearing of the wings 118 when, for example, the clip 102 is drawn proximally over the adapter 110 toward the insertion configuration during the removal of the clip 102 from previously clipped tissue for a repositioning of the clip 102.

Upon a shearing off of the wings 118, the wings 118 are separated from the distal ends 114 of the extending members 112 so that the distal ends 114 may be removed from the insert 162 via the hole 164. In particular, subsequent to the initial distal movement of the extending members 112 relative to the clip 102 which initiates the final deployment of the clip 102, upon shearing off of the wings 118, the extending members 112 may be drawn proximally relative to the clip 102 so that the distal end 114 may be passed distally out of the hole 164. The clip 102 is thus finally deployed, remaining on the body clipped over the target tissue. The wings 118 remain trapped within the recess 170 to prevent any shed parts within the body.

According to an exemplary method for tissue closure utilizing the clipping system 100, the clip 102 may be inserted through a body lumen such as, for example, the GI tract, to a target area within the body lumen via the insertion device 104 which, in one embodiment, may include the endoscope 106. As described above, in the insertion configuration (as shown in Fig. 1), the clip 102 is mounted to the distal end 108 of the endoscope 106 via the adapter 110 so that jaws 120 thereof are separated from one another toward the open configuration. In this insertion configuration, the clip 102 is guided to the target area via the visualization system of the endoscope 106 and positioned over a target tissue. A suction force and/or tissue graspers may be applied through a working channel of the endoscope 106 so that the target tissue may be drawn into the channel 126 of the adapter 110. Thus, when the clip 102 is moved toward the initial deployed configuration by moving the extending members 112 distally relative to the endoscope 10, thereby releasing a tension therealong, the clip 102 is permitted to slide distally along the adapter 110 toward the biased closed configuration. As the clip 102 is moved toward the biased closed configuration, tissue is gripped between the jaws 120 of the clip 102 in the initial deployed configuration.

It will be understood by those of skill in the art that suctioning and/or gripping of the tissue in this initial deployed configuration may obstruct an imaging/optical lens of the endoscope 106 so that the user is unable to visualize and/or confirm whether a desired target tissue has been properly clipped. Thus, the clip 102 may be moved toward the review configuration, as shown in Fig. 5) by drawing the endoscope 106 proximally relative to the clip 102, while the clip 102 remains engaged with the extending members 112. A distance between the adapter 110 and the clip 102 widens a field of view of the endoscope 106 so that the clip 102, and the tissue gripped thereby, may be viewed via the optical/visualization system of the endoscope 106.

If, upon visualization, the user determines that the clip 102 requires an adjustment and/or a repositioning relative to the target tissue, the extending members 112 be translated proximally relative to the endoscope 106 until the clip 102 is moved proximally over the adapter 110, as described above, toward the open insertion configuration. In particular, the endoscope 106 may be moved distally relative to the extending members 112, so that the clip 102 and the adapter 110 are drawn toward one another. As the clip 102 is moved toward the open configuration, the tissue gripped thereby is released, permitting the clip 102 to be repositioned over the target tissue, as desired. The clip 102 may then once again moved toward the initial deployed configuration, and then again toward the review configuration. This process may be repeated, as necessary, until the user is able to visually confirm that the clip 102 has been clipped over the target tissue, as desired.

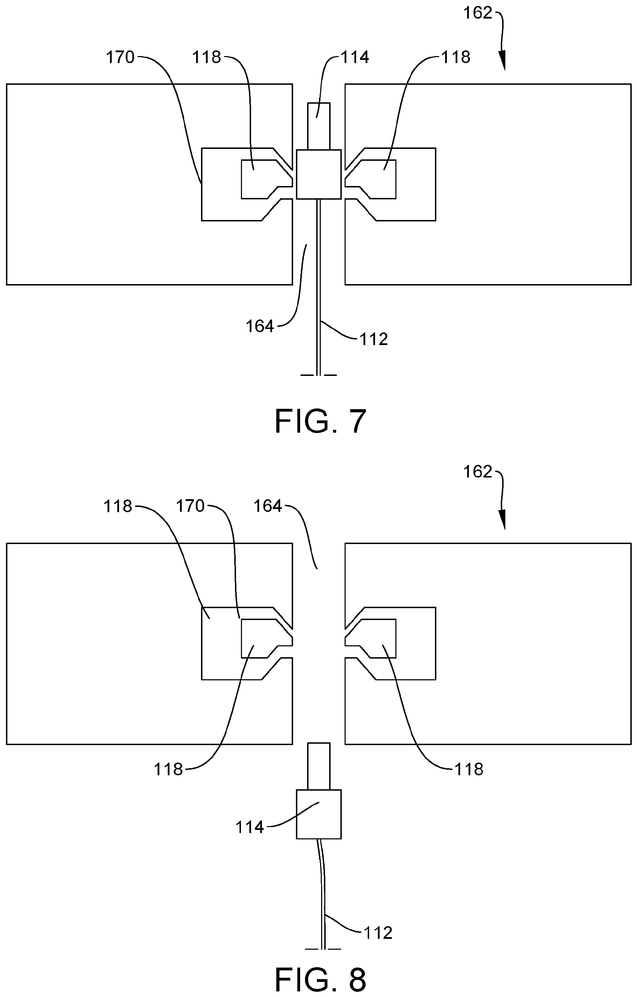

Once the user confirms that the target tissue has been clipped, as desired, the clip 102 may be moved from the review configuration to the final deployed configuration, by releasing the clip 102 from the extending members 112. As described above, in one embodiment, the extending members 112 may be moved distally relative to the clip 102 so that the wings 118 at the distal end 114 of each of the extending members 112 is pressed against the distal surface 171 of the recess 170 of the insert 162 within which it is received, as shown in Fig. 6. The extending members 112 are moved distally relative to the clip 102 until a proximal force exerted on the wings 118 via the distal surface 171 exceeds the predetermined threshold force, causing the cutting edge 172 to shear off the wings 118 at the radially innermost point 132, as shown in Fig. 7.

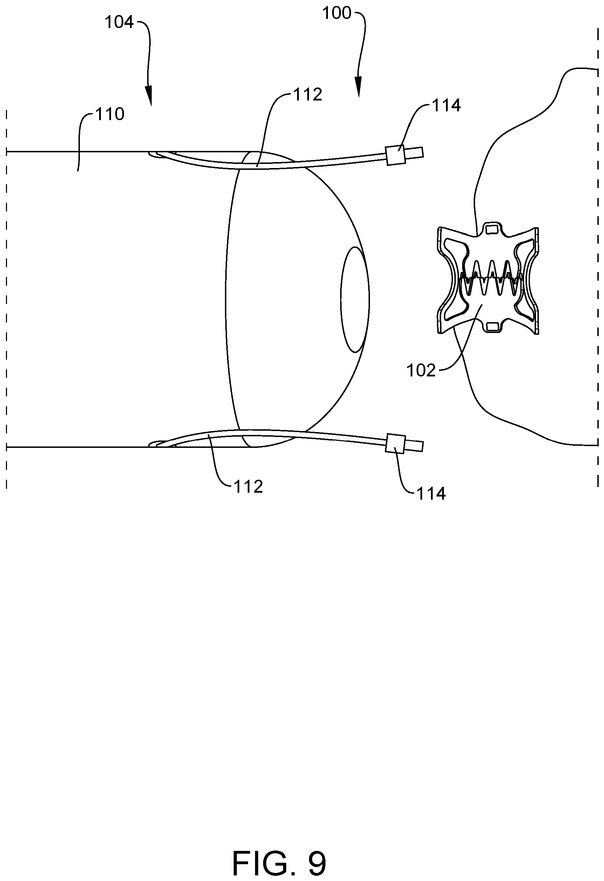

The wings 118 are thus separated from the distal ends 114 so that the extending members 112 may then be drawn distally relative to the clip 102 so that the distal ends 114 are drawn distally out of the hole 164 of the insert 162, as shown in Fig. 8. As described above, the sheared wings 118 remain trapped within the recess 170 to prevent any shed parts during the final deployment process. The extending members 112, and the remaining portions of the insertion device 104 may thereby be proximally removed from the clip 102 and withdrawn from the body, leaving the clip 102 clipped over the target tissue, as shown in Fig. 9.

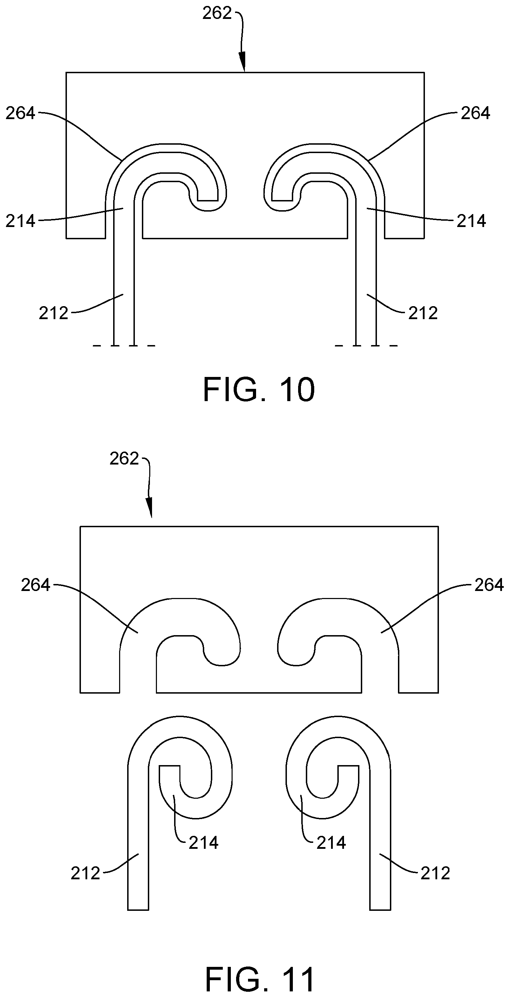

As shown in Figs. 10-11, extending members 212 according to another exemplary embodiment may be releasably engaged to the clip 102, as described above with respect to the system 100, to move the clip 102 between an insertion configuration, an initial deployed configuration, and a review configuration. Upon release of the extending members 212 from the clip 102, the clip 102 may be finally deployed. Similarly to the extending members 112, the extending members 212 may be directly coupled to the opening 156 of the clip 102 or, in another embodiment as shown in the figures, the extending members 212 may be coupled to the clip 102 via an insert 262 received within and affixed to the opening 156 of the clip 102. Rather than a connector including wings that may be sheared off, however, a distal end 214 of each of the extending members 212 is biased toward a curled configuration and is receivable within an opening 264 of the insert 262 in an engaged configuration, as shown in Fig. 10.

In an exemplary embodiment, the opening 264 may extend through the insert 262 along a curve which has a curvature that is smaller than the curvature of the curled distal end 214 so that the distal end 214 engages the opening 264 and permits movement of the clip 102 (and the insert 262) between the insertion configuration, initial deployed configuration, and the review configuration via movement of the extending members 212. In other words, the bias of the curled distal end 214 causes the distal end 214 to grip the curved surface of the opening 264 until a force exerted thereon exceeds a predetermined threshold force.

When it is desired to move the clip 102 toward the final deployed configuration, the extending member 212 are drawn proximally relative to the clip 102 until the force exerted thereon exceeds a predetermined threshold force, causing the curled distal end 214 to unravel so that the distal end 214 may be removed from the opening 264, as shown in Fig. 11. It will be understood by those of skill in the art that while the extending members 112 described above with respect to the system 100 require distal movement of the extending members 112 relative to the clip 102 to release the distal ends 114 from the insert, the extending members 212 are moved proximally relative to the clip 102 to release the distal ends 214 from the openings 264 of the insert 262.

In an exemplary embodiment, at least a portion of the extending members 212 (e.g., the distal ends 214) may be formed of a material biased toward the curled configuration including, for example, nitinol or a spring steel. In one embodiment, to achieve a desired curling of the distal ends 214 and to ensure that the distal ends 214 do not unravel during movement of the clip 102 between the insertion configuration, the initial deployed configuration, and the review configuration, the distal ends 214 may be reinforced with, for example, a compressible rod. It will be understood by those of skill in the art that the force required to release the extending members 212 from the inserts 262 may be adjusted in any of a number of different ways such as, for example, adjusting a level of curling of the distal ends 214, a thickness or material of the distal ends 214, or a geometry of the openings 264 of the inserts 262. In one embodiment, the openings 264 may include angled edges further facilitating a gripping of the surface of the openings 264 via the biased curls of the distal ends 214.

According to another exemplary embodiment, rather than being received within a curved opening 264, the curled distal ends 214 may be wrapped around a post or other geometrical structure within the insert 262. As described above, when the extending members 212 are drawn proximally relative to the clip 102 via a force exceeding a predetermined threshold force, the curled distal ends 214 may unravel to release the post so that the extending members 212 may be removed from the insert 262 to deploy the clip 102. In yet another exemplary embodiment, rather than unraveling by exerting a proximal force on the extending members 212 which exceeds a predetermined threshold force, an oversheath may be moved distally over distal ends 214 of the extending members 212, causing the distal ends 214 to be straightened so that the distal ends 214 may be removed from the insert, thereby releasing the clip 102.

Similarly to the system 100 described above, the extending members 212 may be moved longitudinally relative to the endoscope 106 to move the clip 102 between the insertion configuration, the initial deployed configuration, and the review configuration. It will be understood by those of skill in the art that, during a repositioning of the clip 102, a proximal force exerted on the extending members 212 to move the clip 102 proximally over the adapter 110 from the review configuration back toward the insertion configuration is less than the requisite predetermined threshold force for a final deployment of the clip 102.

In other words, retracting the clip 102 over the adapter 110 will not cause the distal ends 214 to unravel so that the clip 102 may be moved between the insertion configuration, the initial deployed configuration, and the review configuration, as necessary, until the clip 102 is clipped over a target tissue, as desired. Once it is determined that the clip 102 is clipped over the target tissue, extending members 212 may be drawn proximally relative to the clip 102 until the force exerted thereon exceeds the predetermined threshold force, causing the curled distal ends 214 to unravel and be released from the insert 262, thereby deploying the clip 102. Upon release from the insert 262, the extending members 212 (and the remaining portions of the insertion device 104) are withdrawn from the clip 102 and the body, leaving the clip 102 clipped over the target tissue.

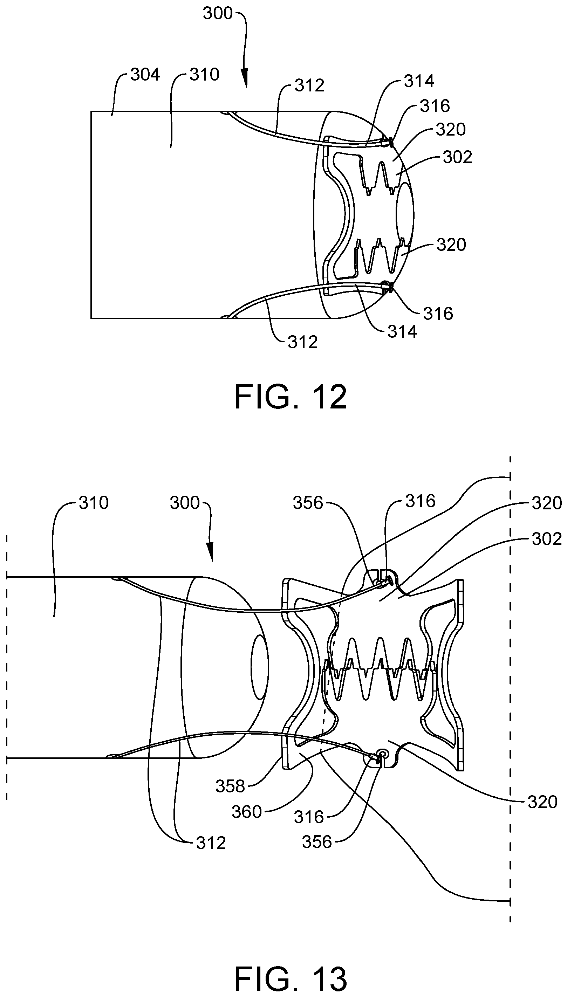

A tissue clipping system 300 according to another exemplary embodiment, as shown in Figs. 12-16, may be substantially similar to the clipping system 100, as described above, comprising a clip 302 coupled to a distal end of an insertion device 304 such as, for example, an endoscope, via an adapter 310 mounted over the distal end of the endoscope. Similarly to the clip 102, the clip 302 is movable relative to the adapter 310 (and the endoscope over which it is mounted) between an insertion configuration (Fig. 12), an initial deployed configuration, and a review configuration (Fig. 13) via extending members 312 coupled to the clip 302. Once it is determined that the clip 302 is clipped over a target tissue, as desired, the extending members 312 may be released from the clip 302 to move the clip from the review configuration toward a final deployed configuration.

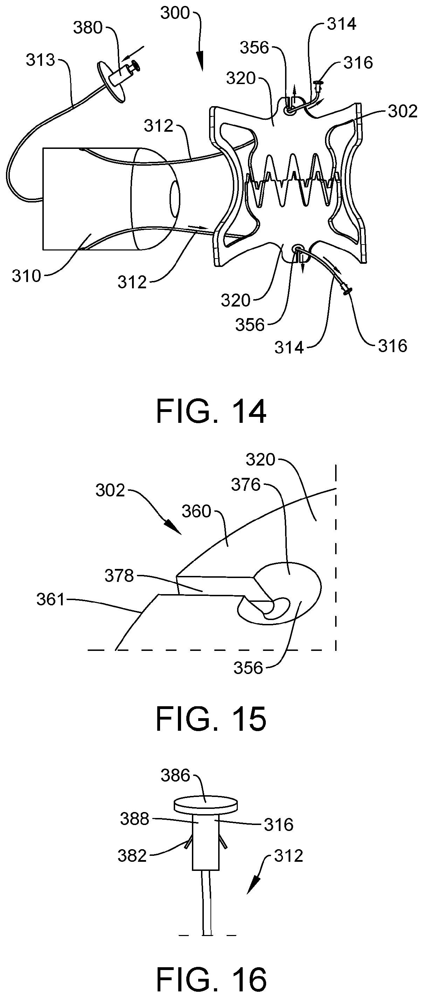

In this embodiment, however, distal ends 314 of the extending members 312 are releasably coupled to the clip 302 via connectors 316 configured to engage openings 356 extending through jaws 320 of the clip 302. The distal ends 314, however, are biased radially away from one another and/or laterally away from a longitudinal axis of the adapter 310 so that when the extending members 312 are moved distally relative to the clip 302 via a force exceeding a predetermined threshold force, the connectors 316 move distally out of a first portion 376 of the openings 356, as shown in Fig. 14, so that the distal ends 314 are permitted to revert to their biased configuration, in which the distal ends 314 are moved away from one another. As the distal ends 314 are moved away from one another, a length of the extending members 312 extending proximally from the connectors 316 are moved laterally relative to a longitudinal axis of the adapter 310, through a second portion 378 of the openings 356 extending between the first portion 376 of the openings 356 and an exterior of the clip 302. The extending members 312 pass through the second portion 378 of the openings 356 so that the extending members 312 are no longer coupled to the clip 302 and the extending members 312 may be withdrawn proximally from the clip 302, leaving the clip 302 clipped over the target tissue in the final deployed configuration.

The clip 302, in this embodiment, may be substantially similar to the clip 102 as described above with respect to the system 100. As shown in Fig. 15, the opening 356 extending through each jaw 320 of the clip 302 from a first surface which faces toward the adapter 310 to a second surface 360 which faces away from the adapter 310, however, may be substantially keyhole shaped, including the first portion 376 and the second portion 378. As described above, the first portion 376 is sized and shaped to correspond to the connector 316 so that the connector 316 may be received and engaged therewithin. The second portion 378 extends from the first portion 376 to an exterior edge 361 of the each of the jaws 320 so that the opening 356 is open to an exterior of the clip 302 via the second portion 378. The second portion 378 may be configured as a slot having a smaller width than the first portion 376 so that the connector 316 cannot be received therewithin. The width of the second portion 378, however, is configured to permit passage of a remaining length of the extending members 312 (e.g., a portion of the extending members 312 extending proximally from the connectors 316) therethrough.

Each of the extending members 312 extends from a proximal end 313 accessible to a user via, for example, an actuator 380 to a distal end 314 including the connector 316. As described above, each connector 316 is sized, shaped and configured to engage the first portion 376 of the opening 356 of a corresponding jaw 320 of the clip 302 via, for example, a friction fit or a snap connection. The distal ends 314 of the extending members 312 are biased radially away from one another so that, the distal ends 314 are constrained toward one another when the connectors 316 are received within the first portions 376 of the clip 302.

In an exemplary embodiment, as shown in Fig. 16, each of the connectors 316 includes a head portion 386 and a shaft 388 extending proximally therefrom. The head portion 386 is configured to be positioned along the second surface 360 of the clip 302 when the shaft 388 is received within the first portion 376. The connector 316 may also include one or more arms 382 extending laterally from the shaft 388, the one or more arms 382 positioned along the shaft 388 so that when the connector 316 is engaged with the first portion 376 of the opening 356, the arms 382 extend along and/or adjacent to the first surface 358 of the clip 302 to prevent the clip 302 from inadvertently disengaging the connector 316 when the clip 302 is pushed against tissue. Thus, each connector 316 may be received within a corresponding one of the openings 356 such that the clip 302 is received between the head portion 386 and the arms 382 thereof.

The system 300 may be utilized to place a clip 302 in a manner substantially similar to the system 100 described above. In particular, while the clip 302 remains coupled to the extending members 312, as described above, the clip 302 may be moved between the insertion configuration, the initial deployed configuration and the review configuration. Similarly to the clip 102, in the insertion configuration, the clip 302 is mounted over the adapter 310 with jaws 320 extending over opposing portions thereof so that tissue may be received therebetween. To move the clip 302 toward the initial deployed configuration, a tension along the extending members 312 may be released so that the clip 302 slides distally off of the adapter toward the biased closed configuration to grip tissue between the jaws 320. The clip 302 may be moved toward the review configuration by drawing the adapter 310 proximally away from the clip 302 to widen a scope of view of the endoscope. The user may then observe whether the clip 302 is clipped over a target tissue, as desired.

As described above with respect to the system 100, the clip 302 may be similarly repositioned by drawing the clip 302 proximally over adapter 310 back toward the insertion configuration. The above-described process may be repeated, moving the clip 302 between the insertion configuration, the initial deployed configuration, and the review configuration, until the clip 302 is determined to be clipped over the target tissue, as desired.

Once the clip 302 is in the review configuration and it is determined that the clip 302 is clipped over the target tissue, as desired, the extending members 312 are moved distally relative to the clip 302 until a force exerted thereon exceeds a predetermined threshold force, which causes the arms 382 to deform, allowing each connector 316 to be moved distally out of the first portion 376 of the corresponding opening 356. As the connectors 316 are moved distally out of the openings 356, the distal ends 314 are permitted to revert to their biased configuration, moving away from one another such that a length of the extending members 312 extending proximally from the connectors 316 passes through the second portions 378 of the openings 356 to an exterior of the clip 302. Thus, the extending members 312 are released from the clip 302 so that the extending members 312, and a remaining portion of the insertion device 304, may be withdrawn from the body, leaving the clip 302 clipped over the target tissue in the final deployed configuration.

Although the exemplary embodiments show and describe the openings 356 of the clip 302 as being directly coupled to the connectors 316 of the extending members 312, it will be understood by those of skill in the art that, similarly to the clip 102, the opening 356 may be configured to receive an insert having the features of the first and second portions 376, 378 so that the connectors 316 may be connected to the clip 302 via an insert affixed within the opening 356.

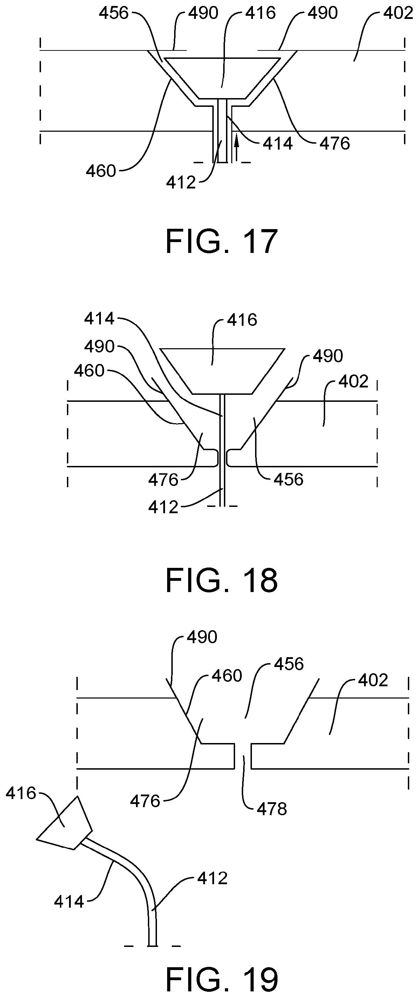

According to an alternate embodiment, as shown in Figs. 17-19, rather than a connector 316 including arms 382 as described above for preventing inadvertent release of the connector 316 from the clip 302, an insert and/or an opening 456 of a clip 402 may include flaps 490 extending over a first portion 476 of the opening 456 along a surface 460 of the clip 402 facing away from an adapter, as shown in Fig. 17. The flaps 490 are configured to deform, as shown in Fig. 18, allowing a connector 416 of a corresponding extending member 412 to pass distally therepast, when a distal force applied to the extending member 412 exceeds a predetermined threshold force. Similarly to the system 300, once the connector 416 is moved distally out of the first portion 476 of the opening 456, distal ends 414 of extending members 412 revert to a biased configuration, moving away from one another so that a length of the extending members proximal of the connecting members 412 pass laterally through a second portion of the openings 456 of the clip 402 to an exterior of the clip 402 so that the extending members 412 may be proximally withdrawn therefrom, as shown in Fig. 19, leaving the clip 402 clipped over a target tissue in its final deployed configuration.

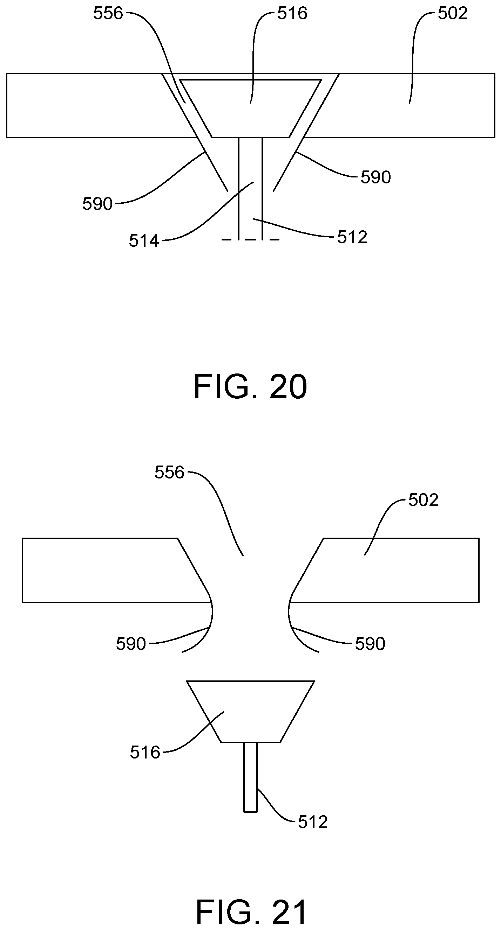

According to yet another alternate embodiment, as shown in Figs. 20-21, flaps 590 may extend over an opening 556 of a clip 502 along a surface 558 facing in an initial configuration toward an adapter over which the clip 502 is mountable. In this embodiment, when the extending member 512 is drawn proximally relative to the clip 502 via a force exceeding a predetermined threshold force, the flaps 590 deform, as shown in Fig. 20, allowing a connector 516 of the extending member 512 to be released from the opening 556 of the clip 502 so that the clip 502 may be moved toward the final deployed configuration.

It will be understood by those of skill in the art that this embodiment does not require the distal ends 514 of extending members 512 to be biased away from one another nor does the openings 556 of the clip 502 nor does it require a slot which opens the opening 556 to an exterior of the clip 502. As described above, once the flap 590 is deformed, the connector 516 is released from the opening 556 so that drawing the extending members 512 proximally away from the clip 502 (as shown in Fig. 21) and out of the body leaves the clip 502 clipped over the target tissue in the final deployed configuration.

It will be apparent to those skilled in the art that various modifications may be made in the present disclosure, without departing from the scope of the claims.

## Claims

1. A clipping system (100, 300) for treating tissue, comprising:
an adapter (110, 310) including a proximal portion configured to be mounted over a distal end of an insertion device and a distal portion extending distally from the proximal portion;
a clip (102, 302, 402, 502) configured to be mounted over the distal portion of the adapter (110, 310), the clip (102, 302, 402, 502) including first and second jaws (120, 320) connected to one another such that the first and second jaws (120, 320) are movable between an insertion configuration, in which the first and second jaws (120, 320) extend about opposing portions of the distal portion of the adapter (110, 310) and are separated from one another to receive tissue therebetween, and an initial deployed configuration, in which the clip (102, 302, 402, 502) is moved distally off of the adapter (110, 310) so that the first and second jaws (120, 320) are drawn toward one another to grip tissue therebetween, the first and second jaws (120, 320) being biased toward the initial deployed configuration; and
a first extending member (112, 312, 412, 512) releasably coupled to the clip (102, 302, 402, 502) and movably connected to the adapter (110, 310), the first extending member (112, 312, 412, 512) including a connector (116, 316, 416, 516) at a distal end thereof configured to releasably engage an opening (156, 356, 456, 556) extending through the first jaw (120, 320) such that a longitudinal movement of the first extending member (112, 312, 412, 512) relative to the adapter (110, 310) moves the clip (102, 302, 402, 502) between the insertion configuration, the initial deployed configuration and toward a review configuration, in which the adapter (110, 310) is permitted to be withdrawn proximally away from the clip (102, 302, 402, 502) while the first extending member (112, 312, 412, 512) remains coupled to the clip (102, 302, 402, 502) to enhance visual observation of the clip (102, 302, 402, 502), the first extending member (112, 312, 412, 512) being operable to retract the clip (102, 302, 402, 502) proximally over the adapter (110, 310) so that the clip (102, 302, 402, 502) is forced open as the clip (102, 302, 402, 502) is retracted over the adapter (110, 310) freeing the clip (102, 302, 402, 502) from tissue on which it had been clipped, the connector including a deformable portion configured to deform to release the first extending member (112, 312, 412, 512) from the first jaw (120, 320) toward a final deployed configuration when the first extending member (112, 312, 412, 512) is moved distally relative to the clip (102, 302, 402, 502) via a force exceeding a predetermined threshold force, the first extending member (112, 312, 412, 512) being proximally withdrawable from the clip (102, 302, 402, 502) upon deformation of the connector.

2. The system (100, 300) of claim 1, wherein the connector (116) includes a pair of wings (118) extending radially away from the distal end of the first extending member (112), the pair of wings (118) configured to shear off of the distal end of the first extending member (112) as the pair of wings (118) are deformed toward the final deployed configuration.

3. The system (100, 300) of claim 2, further comprising an insert (162) configured to be received and affixed within the opening (156) of the first jaw (120, 320), the insert (162) including a hole (164) extending longitudinally therethrough and a recess (170) extending radially outward from the hole (164), the recess (170) sized, shaped and configured to receive the pair of wings (118) therein.

4. The system (100, 300) of claim 2, wherein a radially innermost point at which each of the wings (118) is connected to the distal end of the first extending member (112) has a thickness smaller than a remaining portion of the wings (118).

5. The system (100, 300) of claim 3, wherein a distal surface of the pair of wings (118) includes a tapered portion angled at a non-perpendicular angle relative to a longitudinal axis of the first extending member (112), the tapered portion configured to engage a sharp cutting edge (172) along a surface of the recess (170) when pressed distally thereagainst.

6. The system (100, 300) of any one of claims 1-5, wherein the opening of the first jaw (120, 320) extending through the clip (102, 302, 402, 502) from a first surface facing toward the adapter (110, 310) to a second surface facing away from the adapter (110, 310), the opening (356, 456, 556) including a first portion (376, 476) configured to engage the connector and a second portion (378) configured as a slot extending from the first portion to an exterior edge of the first jaw (120, 320) so that the opening is open to an exterior of the clip (102, 302, 402, 502).

7. The system (100, 300) of claim 6, wherein the connector of the first extending member (312, 412, 512) includes a head portion (386) and a shaft (388) extending proximally therefrom, the shaft (388) being sized, shaped and configured to engage the first portion (376, 476) of the opening (356, 456, 556), a width of the slot being smaller than the shaft (388) to prevent a passage of the connector therethrough.

8. The system (100, 300) of claim 6, wherein the distal end of the first extending member (112, 312, 412, 512) is biased laterally away from a longitudinal axis of the adapter (110, 310) so that, when the connector is received within the first portion of the opening the distal end of the first extending member (112, 312, 412, 512) is constrained toward a laterally inward position, and when the connector is pushed distally out of the first portion of the opening, the distal end reverts towards its biased laterally outward position so that a remaining length of the first extending member (112, 312, 412, 512) extending proximally from the connector is passed through the slot of the second portion of the opening to an exterior of the clip (102, 302, 402, 502).

9. The system (100, 300) of claim 7, wherein the connector including a deformable arm extending laterally from the shaft to engage the first surface of the clip (102, 302, 402, 502) when the shaft is received within the first portion of the opening to move the clip (102, 302, 402, 502) between the insertion configuration, the initial deployed configuration and the review configuration, the deformable arm configured to deform to release the connector from the first portion of the opening when the first extending member (112, 312, 412, 512) is moved distally relative to the clip (102, 302, 402, 502) via a force exceeding the predetermined threshold force.

10. The system (100, 300) of any one of claims 1-9, further comprising a second extending member (112, 312, 412, 512) releasably coupled to the clip (102, 302, 402, 502) and movably connected to the adapter (110, 310), the second extending member (112, 312, 412, 512) including a further connector at a distal end thereof configured to releasably engage an opening extending through the second jaw (120, 320) such that a longitudinal movement of the second extending member (112, 312, 412, 512) relative to the adapter (110, 310) moves the clip (102, 302, 402, 502) between the insertion configuration, the initial deployed configuration and the review configuration, the further connector of the second extending member (112, 312, 412, 512) including a deformable portion configured to deform to release the second extending member (112, 312, 412, 512) from the second jaw (120, 320) toward the final deployed configuration when the second extending member is moved distally relative to the clip (102, 302, 402, 502) via a force exceeding a predetermined threshold force.

11. A clipping system (100, 300) for treating tissue, comprising:
an adapter (110, 310) including a proximal portion configured to be mounted over a distal end of an insertion device and a distal portion extending distally from the proximal portion;
a clip (102, 302, 402, 502) configured to be mounted over the distal portion of the adapter (110, 310), the clip (102, 302, 402, 502) including first and second jaws (120, 320) connected to one another such that the first and second jaws (120, 320) are movable between an insertion configuration, in which the first and second jaws (120, 320) extend about opposing portions of the distal portion of the adapter (110, 310) and are separated from one another to receive tissue therebetween, and an initial deployed configuration, in which the clip (102, 302, 402, 502) is moved distally off of the adapter (110, 310) so that the first and second jaws (120, 320) are drawn toward one another to grip tissue therebetween, the first and second jaws (120, 320) being biased toward the initial deployed configuration; and
a first extending member (212) releasably coupled to the clip (102, 302, 402, 502) and movably connected to the adapter (110, 310), the first extending member (212) including a distal end biased toward a coiled configuration and configured to releasably engage a portion of an opening extending through the first jaw (120, 320) such that a longitudinal movement of the first extending member (212) relative to the adapter (110, 310) moves the clip (102, 302, 402, 502) between the insertion configuration, the initial deployed configuration and toward a review configuration, in which the adapter (110, 310) is permitted to be withdrawn proximally away from the clip (102, 302, 402, 502) while the first extending member (212) remains coupled to the clip (102, 302, 402, 502) to enhance visual observation of the clip (102, 302, 402, 502), the first extending member (212) being operable to retract the clip (102, 302, 402, 502) proximally over the adapter (110, 310) so that the clip (102, 302, 402, 502) is forced open as the clip (102, 302, 402, 502) is retracted over the adapter (110, 310) freeing the clip (102, 302, 402, 502) from tissue on which it had been clipped, the coiled configuration of the first extending member (212) configured to unravel when subject to a force exceeding a predetermined threshold value so that the first extending member (212) is disengaged from the first jaw (120, 320) toward a final deployed configuration.

12. The system (100, 300) of claim 11, further comprising an insert configured to be received and affixed within the opening of the first jaw (120, 320), the insert including a curved opening extending thereinto, the curved opening configured to receive and engage the distal end of the first extending member (212).

13. The system (100, 300) of any one of claims 11-12, wherein the distal end of the first extending member (212) is reinforced with a compressible rod.

14. The system (100, 300) of claim 12, wherein the curved opening includes an angled edge extending therealong.

15. The system (100, 300) of any one of claims 11-14, further comprising a second extending member (212) releasably coupled to the clip (102, 302, 402, 502) and movably connected to the adapter (110, 310), the second extending member (212) including a distal end biased toward a coiled configuration and configured to releasably engage a portion of an opening extending through the second jaw (120, 320) such that a longitudinal movement of the second extending member (212) relative to the adapter (110, 310) moves the clip (102, 302, 402, 502) between the insertion configuration, the initial deployed configuration and the review configuration, the coiled configuration of the second extending member (212) configured to unravel when subject to a force exceeding a predetermined threshold value so that the second extending member (212) is disengaged from the second jaw (120, 320) toward the final deployed configuration.

## Patentansprüche

1. Clipanlagesystem (100, 300) zur Behandlung von Gewebe, das aufweist:
einen Adapter (110, 310) mit einem proximalen Abschnitt, der zur Montage auf einem distalen Ende einer Einführvorrichtung konfiguriert ist, und einem distalen Abschnitt, der sich vom proximalen Abschnitt distal erstreckt;
einen Clip (102, 302, 402, 502), der zur Montage auf dem distalen Abschnitt des Adapters (110, 310) konfiguriert ist, wobei der Clip (102, 302, 402, 502) eine erste und zweite Backe (120, 320) aufweist, die miteinander so verbunden sind, dass die erste und zweite Backe (120, 320) beweglich sind zwischen einer Einführkonfiguration, in der sich die erste und zweite Backe (120, 320) um gegenüberliegende Abschnitte des distalen Abschnitts des Adapters (110, 310) erstrecken und voneinander getrennt sind, um Gewebe dazwischen aufzunehmen, und einer ersten Freisetzkonfiguration, in der der Clip (102, 302, 402, 502) vom Adapter (110, 310) distal weg bewegt ist, so dass die erste und zweite Backe (120, 320) zueinander gezogen sind, um Gewebe dazwischen zu ergreifen, wobei die erste und zweite Backe (120, 320) in die erste Freisetzkonfiguration vorgespannt sind; und
ein erstes Ausfahrelement (112, 312, 412, 512), das mit dem Clip (102, 302, 402, 502) lösbar gekoppelt und mit dem Adapter (110, 310) beweglich verbunden ist, wobei das erste Ausfahrelement (112, 312, 412, 512) einen Verbinder (116, 316, 416, 516) an einem distalen Ende davon aufweist, der so konfiguriert ist, dass er einen lösbaren Eingriff mit einer Öffnung (156, 356, 456, 556) herstellt, die sich durch die erste Backe (120, 320) erstreckt, so dass eine Längsbewegung des ersten Ausfahrelements (112, 312, 412, 512) relativ zum Adapter (110, 310) den Clip (102, 302, 402, 502) zwischen der Einführkonfiguration, der ersten Freisetzkonfiguration und in eine Überprüfungskonfiguration bewegt, in der der Adapter (110, 310) proximal weg vom Clip (102, 302, 402, 502) zurückgezogen werden kann, während das erste Ausfahrelement (112, 312, 412, 512) mit dem Clip (102, 302, 402, 502) gekoppelt bleibt, um eine visuelle Beobachtung des Clips (102, 302, 402, 502) zu verstärken, wobei das erste Ausfahrelement (112, 312, 412, 512) bedienbar ist, um den Clip (102, 302, 402, 502) über den Adapter (110, 310) proximal einzufahren, so dass der Clip (102, 302, 402, 502) aufgezwungen wird, wenn der Clip (102, 302, 402, 502) über den Adapter (110, 310) zurückgezogen wird, was den Clip (102, 302, 402, 502) von Gewebe befreit, auf dem er angelegt war, wobei der Verbinder einen verformbaren Abschnitt aufweist, der so konfiguriert ist, dass er sich verformt, um das erste Ausfahrelement (112, 312, 412, 512) von der ersten Backe (120, 320) in eine endgültige Freisetzkonfiguration zu lösen, wenn das erste Ausfahrelement (112, 312, 412, 512) relativ zum Clip (102, 302, 402, 502) über eine Kraft distal bewegt ist, die eine vorbestimmte Schwellenkraft übersteigt, wobei das erste Ausfahrelement (112, 312, 412, 512) vom Clip (102, 302, 402, 502) bei Verformung des Verbinders proximal zurückziehbar ist.

2. System (100, 300) nach Anspruch 1, wobei der Verbinder (116) ein Paar Flügel (118) aufweist, die sich vom distalen Ende des ersten Ausfahrelements (112) radial weg erstrecken, wobei das Paar Flügel (118) so konfiguriert ist, dass es vom distalen Ende des ersten Ausfahrelements (112) abschert, wenn das Paar Flügel (118) in die endgültige Freisetzkonfiguration verformt wird.

3. System (100, 300) nach Anspruch 2, ferner mit einem Einsatz (162), der so konfiguriert ist, dass er in der Öffnung (156) der ersten Backe (120, 320) aufgenommen und befestigt ist, wobei der Einsatz (162) aufweist: ein Loch (164), das sich längs durch ihn erstreckt, und eine Aussparung (170), die sich vom Loch (164) radial nach außen erstreckt, wobei die Aussparung (170) so bemessen, geformt und konfiguriert ist, dass sie das Paar Flügel (118) darin aufnimmt.

4. System (100, 300) nach Anspruch 2, wobei ein radial innerster Punkt, an dem jeder der Flügel (118) mit dem distalen Ende des ersten Ausfahrelements (112) verbunden ist, eine Dicke hat, die kleiner ist als ein restlicher Abschnitt der Flügel (118).

5. System (100, 300) nach Anspruch 3, wobei eine distale Oberfläche des Paars Flügel (118) einen zulaufenden Abschnitt aufweist, der in einem nicht senkrechten Winkel relativ zu einer Längsachse des ersten Ausfahrelements (112) abgewinkelt ist, wobei der zulaufende Abschnitt so konfiguriert ist, dass er einen Eingriff mit einer scharfen Schneidkante (172) entlang einer Oberfläche der Aussparung (170) herstellt, wenn er distal daran gedrückt wird.

6. System (100, 300) nach einem der Ansprüche 1 bis 5, wobei sich die Öffnung der ersten Backe (120, 320) durch den Clip (102, 302, 402, 502) von einer zum Adapter (110, 310) weisenden ersten Oberfläche zu einer vom Adapter (110, 310) wegweisenden zweiten Oberfläche erstreckt, wobei die Öffnung (356, 456, 556) aufweist: einen ersten Abschnitt (376, 476), der so konfiguriert ist, dass er einen Eingriff mit dem Verbinder herstellt, und einen zweiten Abschnitt (378), der als Schlitz konfiguriert ist, der sich vom ersten Abschnitt zu einer Außenkante der ersten Backe (120, 320) erstreckt, so dass die Öffnung zu einer Außenseite des Clips (102, 302, 402, 502) offen ist.

7. System (100, 300) nach Anspruch 6, wobei der Verbinder des ersten Ausfahrelements (312, 412, 512) einen Kopfabschnitt (386) und einen sich davon proximal erstreckenden Schaft (388) aufweist, wobei der Schaft (388) so bemessen, geformt und konfiguriert ist, dass er einen Eingriff mit dem ersten Abschnitt (376, 476) der Öffnung (356, 456, 556) herstellt, wobei eine Breite des Schlitzes kleiner ist als der Schaft (388), um einen Durchgang des Verbinders durch ihn hindurch zu verhindern.

8. System (100, 300) nach Anspruch 6, wobei das distale Ende des ersten Ausfahrelements (112, 312, 412, 512) von einer Längsachse des Adapters (110, 310) lateral weg vorgespannt ist, so dass bei Aufnahme des Verbinders im ersten Abschnitt der Öffnung das distale Ende des ersten Ausfahrelements (112, 312, 412, 512) in eine Lateraleinwärtsposition eingespannt ist und bei distal ausgeschobenem Verbinder aus dem ersten Abschnitt der Öffnung das distale Ende in seine lateral nach außen vorgespannte Position zurückkehrt, so dass eine Restlänge des ersten Ausfahrelements (112, 312, 412, 512), das sich vom Verbinder proximal erstreckt, durch den Schlitz des zweiten Abschnitts der Öffnung zu einer Außenseite des Clips (102, 302, 402, 502) geführt ist.

9. System (100, 300) nach Anspruch 7, wobei der Verbinder einen verformbaren Arm aufweist, der sich vom Schaft lateral erstreckt, um einen Eingriff mit der ersten Oberfläche des Clips (102, 302, 402, 502) herzustellen, wenn der Schaft im ersten Abschnitt der Öffnung aufgenommen ist, um den Clip (102, 302, 402, 502) zwischen der Einführkonfiguration, der ersten Freisetzkonfiguration und der Überprüfungskonfiguration zu bewegen, wobei der verformbare Arm so konfiguriert ist, dass er sich verformt, um den Verbinder vom ersten Abschnitt der Öffnung zu lösen, wenn das erste Ausfahrelement (112, 312, 412, 512) relativ zum Clip (102, 302, 402, 502) über eine Kraft distal bewegt ist, die die vorbestimmte Schwellenkraft übersteigt.

10. System (100, 300) nach einem der Ansprüche 1 bis 9, ferner mit einem zweiten Ausfahrelement (112, 312, 412, 512), das mit dem Clip (102, 302, 402, 502) lösbar gekoppelt und mit dem Adapter (110, 310) beweglich verbunden ist, wobei das zweite Ausfahrelement (112, 312, 412, 512) einen weiteren Verbinder an einem distalen Ende davon aufweist, der so konfiguriert ist, dass er einen lösbaren Eingriff mit einer Öffnung herstellt, die sich durch die zweite Backe (120, 320) erstreckt, so dass eine Längsbewegung des zweiten Ausfahrelements (112, 312, 412, 512) relativ zum Adapter (110, 310) den Clip (102, 302, 402, 502) zwischen der Einführkonfiguration, der ersten Freisetzkonfiguration und der Überprüfungskonfiguration bewegt, wobei der weitere Verbinder des zweiten Ausfahrelements (112, 312, 412, 512) einen verformbaren Abschnitt aufweist, der so konfiguriert ist, dass er sich verformt, um das zweite Ausfahrelement (112, 312, 412, 512) von der zweiten Backe (120, 320) in die endgültige Freisetzkonfiguration zu lösen, wenn das zweite Ausfahrelement relativ zum Clip (102, 302, 402, 502) über eine Kraft distal bewegt ist, die eine vorbestimmte Schwellenkraft übersteigt.

11. Clipanlagesystem (100, 300) zur Behandlung von Gewebe, das aufweist:
einen Adapter (110, 310) mit einem proximalen Abschnitt, der so konfiguriert ist, dass er über einem distalen Ende einer Einführvorrichtung angebaut ist, und einem distalen Abschnitt, der sich vom proximalen Abschnitt distal erstreckt;
einen Clip (102, 302, 402, 502), der so konfiguriert ist, dass er über dem distalen Abschnitt des Adapters (110, 310) angebaut ist, wobei der Clip (102, 302, 402, 502) eine erste und zweite Backe (120, 320) aufweist, die miteinander so verbunden sind, dass die erste und zweite Backe (120, 320) beweglich sind zwischen einer Einführkonfiguration, in der sich die erste und zweite Backe (120, 320) um gegenüberliegende Abschnitte des distalen Abschnitts des Adapters (110, 310) erstrecken und voneinander getrennt sind, um Gewebe dazwischen aufzunehmen, und einer ersten Freisetzkonfiguration, in der der Clip (102, 302, 402, 502) vom Adapter (110, 310) distal weg bewegt ist, so dass die erste und zweite Backe (120, 320) zueinander gezogen sind, um Gewebe dazwischen zu ergreifen, wobei die erste und zweite Backe (120, 320) in die erste Freisetzkonfiguration vorgespannt sind; und
ein erstes Ausfahrelement (212), das mit dem Clip (102, 302, 402, 502) lösbar gekoppelt und mit dem Adapter (110, 310) beweglich verbunden ist, wobei das erste Ausfahrelement (212) ein distales Ende aufweist, das in eine gewickelte Konfiguration vorgespannt und so konfiguriert ist, dass es einen lösbaren Eingriff mit einem Abschnitt einer Öffnung herstellt, die sich durch die erste Backe (120, 320) erstreckt, so dass eine Längsbewegung des ersten Ausfahrelements (212) relativ zum Adapter (110, 310) den Clip (102, 302, 402, 502) zwischen der Einführkonfiguration, der ersten Freisetzkonfiguration und in eine Überprüfungskonfiguration bewegt, in der der Adapter (110, 310) weg vom Clip (102, 302, 402, 502) proximal zurückgezogen werden kann, während das erste Ausfahrelement (212) mit dem Clip (102, 302, 402, 502) gekoppelt bleibt, um eine visuelle Beobachtung des Clips (102, 302, 402, 502) zu verstärken, wobei das erste Ausfahrelement (212) bedienbar ist, um den Clip (102, 302, 402, 502) über den Adapter (110, 310) proximal einzufahren, so dass der Clip (102, 302, 402, 502) aufgedrückt wird, wenn der Clip (102, 302, 402, 502) über den Adapter (110, 310) eingefahren wird, was den Clip (102, 302, 402, 502) von Gewebe befreit, auf dem er angelegt war, wobei die gewickelte Konfiguration des ersten Ausfahrelements (212) so konfiguriert ist, dass sie sich auseinanderwickelt, wenn sie einer Kraft unterliegt, die einen vorbestimmten Schwellenwert übersteigt, so dass das erste Ausfahrelement (212) aus dem Eingriff mit der ersten Backe (120, 320) in eine endgültige Freisetzkonfiguration gelöst wird.

12. System (100, 300) nach Anspruch 11, ferner mit einem Einsatz, der so konfiguriert ist, dass er in der Öffnung der ersten Backe (120, 320) aufgenommen und befestigt ist, wobei der Einsatz eine gekrümmte Öffnung aufweist, die sich in ihn erstreckt, und die gekrümmte Öffnung so konfiguriert ist, dass sie das distale Ende des ersten Ausfahrelements (212) aufnimmt und einen Eingriff damit herstellt.

13. System (100, 300) nach einem der Ansprüche 11 bis 12, wobei das distale Ende des ersten Ausfahrelements (212) mit einem komprimierbaren Stab verstärkt ist.

14. System (100, 300) nach Anspruch 12, wobei die gekrümmte Öffnung eine sich daran entlang erstreckende abgewinkelte Kante aufweist.

15. System (100, 300) nach einem der Ansprüche 11 bis 14, ferner mit einem zweiten Ausfahrelement (212), das mit dem Clip (102, 302, 402, 502) lösbar gekoppelt und mit dem Adapter (110, 310) beweglich verbunden ist, wobei das zweite Ausfahrelement (212) ein distales Ende aufweist, das in eine gewickelte Konfiguration vorgespannt und so konfiguriert ist, dass es einen lösbaren Eingriff mit einem Abschnitt einer Öffnung herstellt, die sich durch die zweite Backe (120, 320) erstreckt, so dass eine Längsbewegung des zweiten Ausfahrelements (212) relativ zum Adapter (110, 310) den Clip (102, 302, 402, 502) zwischen der Einführkonfiguration, der ersten Freisetzkonfiguration und der Überprüfungskonfiguration bewegt, wobei die gewickelte Konfiguration des zweiten Ausfahrelements (212) so konfiguriert ist, dass sie sich auseinanderwickelt, wenn sie einer Kraft unterliegt, die einen vorbestimmten Schwellenwert übersteigt, so dass das zweite Ausfahrelement (212) aus dem Eingriff mit der zweiten Backe (120, 320) in die endgültige Freisetzkonfiguration gelöst wird.

## Revendications

1. Système de clippage (100, 300) pour traiter un tissu, comprenant :
un adaptateur (110, 310) comprenant une partie proximale configurée pour être montée sur une extrémité distale d'un dispositif d'insertion et une partie distale s'étendant de manière distale depuis la partie proximale ;
un clip (102, 302, 402, 502) configuré pour être monté sur la partie distale de l'adaptateur (110, 310), le clip (102, 302, 402, 502) comprenant des première et deuxième mâchoires (120, 320) reliées l'une à l'autre de sorte que les première et deuxième mâchoires (120, 320) sont mobiles entre une configuration d'insertion, dans laquelle les première et deuxième mâchoires (120, 320) s'étendent autour de parties opposées de la partie distale de l'adaptateur (110, 310) et sont séparées l'une de l'autre pour recevoir du tissu entre elles, et une configuration initiale déployée, dans laquelle le clip (102, 302, 402, 502) est déplacé de manière distale hors de l'adaptateur (110, 310) de sorte que les première et deuxième mâchoires (120, 320) sont rapprochées l'une de l'autre pour saisir du tissu entre elles, les première et deuxième mâchoires (120, 320) étant sollicitées vers la configuration initiale déployée ; et
un premier élément d'extension (112, 312, 412, 512) accouplé de manière amovible au clip (102, 302, 402, 502) et relié de manière mobile à l'adaptateur (110, 310), le premier élément d'extension (112, 312, 412, 512) comprenant un connecteur (116, 316, 416, 516) au niveau de son extrémité distale configuré pour mettre en prise de manière amovible une ouverture (156, 356, 456, 556) s'étendant à travers la première mâchoire (120, 320) de sorte qu'un mouvement longitudinal du premier élément d'extension (112, 312, 412, 512) par rapport à l'adaptateur (110, 310) déplace le clip (102, 302, 402, 502) entre la configuration d'insertion, la configuration initiale déployée et vers une configuration de contrôle, dans laquelle l'adaptateur (110, 310) est autorisé à être retiré de manière proximale à l'opposé du clip (102, 302, 402, 502) tandis que le premier élément d'extension (112, 312, 412, 512) reste couplé au clip (102, 302, 402, 502) afin d'améliorer l'observation visuelle du clip (102, 302, 402, 502), le premier élément d'extension (112, 312, 412, 512) étant apte à rétracter le clip (102, 302, 402, 502) de manière proximale sur l'adaptateur (110, 310) de sorte que le clip (102, 302, 402, 502) est forcé à s'ouvrir au fur et à mesure que le clip (102, 302, 402, 502) est rétracté sur l'adaptateur (110, 310), libérant le clip (102, 302, 402, 502) du tissu sur lequel il avait été clippé, le connecteur comprenant une partie déformable configurée pour se déformer afin de libérer le premier élément d'extension (112, 312, 412, 512) de la première mâchoire (120, 320) vers une configuration déployée finale lorsque le premier élément d'extension (112, 312, 412, 512) est déplacé de manière distale par rapport au clip (102, 302, 402, 502) au moyen d'une force dépassant une force de seuil prédéterminée, le premier élément d'extension (112, 312, 412, 512) pouvant être retiré de manière proximale du clip (102, 302, 402, 502) après déformation du connecteur.

2. Système (100, 300) selon la revendication 1, dans lequel le connecteur (116) comprend une paire d'ailettes (118) s'étendant radialement à l'opposé de l'extrémité distale du premier élément d'extension (112), la paire d'ailettes (118) étant configurée pour se détacher de l'extrémité distale du premier élément d'extension (112) au fur et à mesure que la paire d'ailettes (118) est déformée vers la configuration déployée finale.

3. Système (100, 300) selon la revendication 2, comprenant en outre un insert (162) configuré pour être reçu et fixé dans l'ouverture (156) de la première mâchoire (120, 320), l'insert (162) comprenant un trou (164) s'étendant longitudinalement à travers celui-ci et un évidement (170) s'étendant radialement vers l'extérieur à partir du trou (164), l'évidement (170) étant dimensionné, formé et configuré pour recevoir la paire d'ailettes (118) à l'intérieur de ce dernier.

4. Système (100, 300) selon la revendication 2, dans lequel le point radialement le plus interne auquel chacune des ailettes (118) est reliée à l'extrémité distale du premier élément d'extension (112) présente une épaisseur inférieure à celle d'une partie résiduelle des ailettes (118).

5. Système (100, 300) selon la revendication 3, dans lequel une surface distale de la paire d'ailettes (118) comprend une partie progressivement rétrécie inclinée selon un angle non perpendiculaire par rapport à un axe longitudinal du premier élément d'extension (112), la partie progressivement rétrécie étant configurée pour mettre en prise une arête de coupe vive (172) le long d'une surface de l'évidement (170) lorsqu'elle est comprimée de manière distale contre cette dernière.

6. Système (100, 300) selon l'une quelconque des revendications 1 à 5, dans lequel l'ouverture de la première mâchoire (120, 320) s'étendant à travers le clip (102, 302, 402, 502) d'une première surface faisant face à l'adaptateur (110, 310) vers une deuxième surface orientée à l'opposé de l'adaptateur (110, 310), l'ouverture (356, 456, 556) comprenant une première partie (376, 476) configurée pour mettre en prise le connecteur et une deuxième partie (378) configurée comme une fente s'étendant de la première partie vers un bord extérieur de la première mâchoire (120, 320) de sorte que l'ouverture est ouverte vers un extérieur du clip (102, 302, 402, 502).

7. Système (100, 300) selon la revendication 6, dans lequel le connecteur du premier élément d'extension (312, 412, 512) comprend une partie tête (386) et une tige (388) s'étendant de manière proximale à partir de cette dernière, la tige (388) étant dimensionnée, formée et configurée pour mettre en prise la première partie (376, 476) de l'ouverture (356, 456, 556), une largeur de la fente étant inférieure à celle de la tige (388) afin d'empêcher le passage du connecteur à travers cette dernière.

8. Système (100, 300) selon la revendication 6, dans lequel l'extrémité distale du premier élément d'extension (112, 312, 412, 512) est sollicitée latéralement à l'opposé d'un axe longitudinal de l'adaptateur (110, 310) de sorte que, lorsque le connecteur est reçu dans la première partie de l'ouverture, l'extrémité distale du premier élément d'extension (112, 312, 412, 512) est contrainte vers une position latéralement vers l'intérieur, et lorsque le connecteur est poussé de manière distale hors de la première partie de l'ouverture, l'extrémité distale revient vers sa position sollicitée latéralement vers l'extérieur de sorte qu'une longueur résiduelle du premier élément d'extension (112, 312, 412, 512) s'étendant de manière proximale à partir du connecteur passe à travers la fente de la deuxième partie de l'ouverture vers un extérieur du clip (102, 302, 402, 502).

9. Système (100, 300) selon la revendication 7, dans lequel le connecteur comprend un bras déformable s'étendant latéralement depuis la tige pour mettre en prise la première surface du clip (102, 302, 402, 502) lorsque la tige est reçue dans la première partie de l'ouverture afin de déplacer le clip (102, 302, 402, 502) entre la configuration d'insertion, la configuration initiale déployée et la configuration de contrôle, le bras déformable étant configuré pour se déformer afin de libérer le connecteur de la première partie de l'ouverture lorsque le premier élément d'extension (112, 312, 412, 512) est déplacé de manière distale par rapport au clip (102, 302, 402, 502) au moyen d'une force dépassant la force de seuil prédéterminée.

10. Système (100, 300) selon l'une quelconque des revendications 1 à 9, comprenant en outre un deuxième élément d'extension (112, 312, 412, 512) couplé de manière amovible au clip (102, 302, 402, 502) et relié de manière mobile à l'adaptateur (110, 310), le deuxième élément d'extension (112, 312, 412, 512) comprenant un connecteur supplémentaire au niveau de son extrémité distale configuré pour mettre en prise de manière amovible une ouverture s'étendant à travers la deuxième mâchoire (120, 320) de sorte qu'un mouvement longitudinal du deuxième élément d'extension (112, 312, 412, 512) par rapport à l'adaptateur (110, 310) déplace le clip (102, 302, 402, 502) entre la configuration d'insertion, la configuration initiale déployée et la configuration de contrôle, le connecteur supplémentaire du deuxième élément d'extension (112, 312, 412, 512) comprenant une partie déformable configurée pour se déformer afin de libérer le deuxième élément d'extension (112, 312, 412, 512) de la deuxième mâchoire (120, 320) vers la configuration déployée finale lorsque le deuxième élément d'extension est déplacé de manière distale par rapport au clip (102, 302, 402, 502) au moyen d'une force dépassant une force de seuil prédéterminée.

11. Système de clippage (100, 300) pour traiter un tissu, comprenant :
un adaptateur (110, 310) comprenant une partie proximale configurée pour être montée sur une extrémité distale d'un dispositif d'insertion et une partie distale s'étendant de manière distale depuis la partie proximale ;
un clip (102, 302, 402, 502) configuré pour être monté sur la partie distale de l'adaptateur (110, 310), le clip (102, 302, 402, 502) comprenant des première et deuxième mâchoires (120, 320) reliées l'une à l'autre de sorte que les première et deuxième mâchoires (120, 320) sont mobiles entre une configuration d'insertion, dans laquelle les première et deuxième mâchoires (120, 320) s'étendent autour de parties opposées de la partie distale de l'adaptateur (110, 310) et sont séparées l'une de l'autre pour recevoir du tissu entre elles, et une configuration initiale déployée, dans laquelle le clip (102, 302, 402, 502) est déplacé de manière distale hors de l'adaptateur (110, 310) de sorte que les première et deuxième mâchoires (120, 320) sont rapprochées l'une de l'autre pour saisir du tissu entre elles, les première et deuxième mâchoires (120, 320) étant sollicitées vers la configuration initiale déployée ; et
un premier élément d'extension (212) couplé de manière amovible au clip (102, 302, 402, 502) et relié de manière mobile à l'adaptateur (110, 310), le premier élément d'extension (212) comprenant une extrémité distale sollicitée vers une configuration enroulée et configurée pour mettre en prise de manière amovible une partie d'une ouverture s'étendant à travers la première mâchoire (120, 320) de sorte qu'un mouvement longitudinal du premier élément d'extension (212) par rapport à l'adaptateur (110, 310) déplace le clip (102, 302, 402, 502) entre la configuration d'insertion, la configuration initiale déployée et vers une configuration de contrôle, dans laquelle l'adaptateur (110, 310) est autorisé à être retiré de manière proximale à l'opposé du clip (102, 302, 402, 502) tandis que le premier élément d'extension (212) reste couplé au clip (102, 302, 402, 502) afin d'améliorer l'observation visuelle du clip (102, 302, 402, 502), le premier élément d'extension (212) étant apte à rétracter le clip (102, 302, 402, 502) de manière proximale sur l'adaptateur (110, 310) de sorte que le clip (102, 302, 402, 502) est forcé à s'ouvrir au fur et à mesure que le clip (102, 302, 402, 502) est rétracté sur l'adaptateur (110, 310), libérant le clip (102, 302, 402, 502) du tissu sur lequel il avait été clippé, la configuration enroulée du premier élément d'extension (212) étant configurée pour se dérouler lorsqu'elle est soumise à une force dépassant une valeur de seuil prédéterminée de sorte que le premier élément d'extension (212) est dégagé de la première mâchoire (120, 320) vers une configuration déployée finale.

12. Système (100, 300) selon la revendication 11, comprenant en outre un insert configuré pour être reçu et fixé dans l'ouverture de la première mâchoire (120, 320), l'insert comprenant une ouverture courbe s'étendant à l'intérieur de ce dernier, l'ouverture courbe étant configurée pour recevoir et mettre en prise l'extrémité distale du premier élément d'extension (212).

13. Système (100, 300) selon l'une quelconque des revendications 11 à 12, dans lequel l'extrémité distale du premier élément d'extension (212) est renforcée par une tige compressible.

14. Système (100, 300) selon la revendication 12, dans lequel l'ouverture courbe comprend un bord incliné s'étendant le long de cette dernière.

15. Système (100, 300) selon l'une quelconque des revendications 11 à 14, comprenant en outre un deuxième élément d'extension (212) couplé de manière amovible au clip (102, 302, 402, 502) et relié de manière mobile à l'adaptateur (110, 310), le deuxième élément d'extension (212) comprenant une extrémité distale sollicitée vers une configuration enroulée et configurée pour mettre en prise de manière amovible une partie d'une ouverture s'étendant à travers la deuxième mâchoire (120, 320) de sorte qu'un mouvement longitudinal du deuxième élément d'extension (212) par rapport à l'adaptateur (110, 310) déplace le clip (102, 302, 402, 502) entre la configuration d'insertion, la configuration initiale déployée et la configuration de contrôle, la configuration enroulée du deuxième élément d'extension (212) étant configurée pour se dérouler lorsqu'elle est soumise à une force dépassant une valeur de seuil prédéterminée de sorte que le deuxième élément d'extension (212) est dégagé de la deuxième mâchoire (120, 320) vers la configuration déployée finale.
